# EUROPÄISCHE PATENTSCHRIFT

(11) **EP 2 682 604 B1**
(45) Veröffentlichungstag und Bekanntmachung des Hinweises auf die Patenterteilung: **11.10.2017**
(21) Anmeldenummer: 13174617.4
(22) Anmeldetag: 02.07.2013
(51) Int. Cl.: F04B 43/12, A61M 5/142

(54) **Schlauchrollenpumpe mit einem auto-orientierbaren und -verriegelbaren Rotor sowie medizinisches Gerät zur extrakorporalen Blutbehandlung mit Schlauchrollenpumpe**
Hose reel pump with an automatically oriented and lockable rotor and medical device for extracorporeal blood treatment with hose reel pump
Pompe à rouleau tubulaire doté d'un rotor auto-orientable et auto-verrouillable et appareil médical pour le traitement extracorporel du sang doté d'une pompe à rouleau tubulaire

(30) Priorität: 03.07.2012 DE 102012105913
(43) Veröffentlichungstag der Anmeldung: 08.01.2014
(73) Patentinhaber: B. Braun Avitum AG, 34212 Melsungen (DE)
(72) Erfinder: Schäfer, Oliver, 36286 Neuenstein (DE)
(74) Vertreter: Winter, Brandl, Fürniss, Hübner, Röss, Kaiser, Polte - Partnerschaft mbB

(56) Entgegenhaltungen:
- DE-A1-102007 020 573
- DE-U1- 8 500 320
- US-A- 3 737 257
- US-A- 4 558 996
- US-A- 5 062 775

## Beschreibung

Die Erfindung betrifft eine Schlauchrollenpumpe für ein medizinisches Gerät zur extrakorporalen Blutbehandlung mit einem Pumpengehäuse, das eine gebogene Lauffläche und einen innerhalb der Lauffläche drehbaren Rotor aufweist, wobei ein Schlauchsegment zwischen die Lauffläche und den Rotor einbringbar ist. Der Rotor ist mit einer Wellenaufnahme auf einer Antriebswelle der Schlauchrollenpumpe anbringbar, und es ist wenigstens ein Verriegelungselement zur axialen Sicherung und/oder Drehkopplung des Rotors auf der Antriebswelle vorgesehen.

Die Erfindung betrifft ferner ein medizinisches Gerät zur extrakorporalen Blutbehandlung mit einer solchen Schlauchrollenpumpe.

In medizinischen Geräten zur extrakorporalen Blutbehandlung (Dialyse) werden oftmals Schlauchrollenpumpen eingesetzt, welche das entnommene Blut des Patienten zu einem Dialysator und zum Patienten zurückfördern. Solche Schlauchrollenpumpen arbeiten peristaltisch, wobei ein schlaufenförmiges Schlauchsegment an einer entsprechend gebogenen Lauffläche des Pumpengehäuses anliegt. Ein innerhalb der Lauffläche liegender Rotor der Pumpe bewegt sich dann mit seinen Außenkanten bzw. daran angebrachten Rollen entlang des Schlauchsegments, wobei es den Schlauch lokal eindrückt und so mit den elastischen Materialeigenschaften des Schlauchsegments eine Blutförderung durch das Schlauchsegment ermöglicht. Dafür wird das Blut dem Schlauchsegment über einen ersten Anschluss zugeführt und über einen weiteren Anschluss am anderen Ende des Schlauchsegments wieder abgeführt. Das Schlauchsegment bildet so beispielsweise zusammen mit den zu- und abführenden Leitungen und mehreren Luftfängern ein sogenanntes Überleitsystem, mit dem Blut des Patienten zu einem Dialysator und zum Patienten zurückgefördert wird.

Beispielsweise offenbart die Offenlegungsschrift DE 10 2007 020 573 A1 eine solche Schlauchrollenpumpe mit einem Stator, einem Rotor und einem Rotorantrieb, bei der zwischen den Rotor und die Schlauchrollenbahn des Stators ein Schlauch eingelegt wird. Durch die Rotation des Rotors und damit die Umlaufbewegung von Schlauchrollen wird der Schlauch jeweils gegen die Schlauchrollenbahn des Stators gedrückt, wodurch Flüssigkeit durch den Schlauch gepumpt wird.

Auch die Patentschrift US 7,547,200 B2 offenbart eine solche peristaltische Pumpe mit einem Rotor und Rollen, welche einen eingelegten Schlauch gegen eine halbkreisförmige Schlauchrollenbahn drücken. Dabei hat die Rollenbahn an einem Ende eine abgeschrägte Kante, um den Schlauch aufzunehmen, der an einem auswechselbaren Einsatz angebracht ist.

Das Gebrauchsmuster DE 85 00 320 U1 offenbart eine solche Peristaltische Pumpe mit Rücklaufsperre. Während der Rotor in der ersten Arbeitsstellung koaxial an die Welle gekoppelt ist, besteht die Möglichkeit den Rotor in der zweiten Arbeitsstellung manuell zu betätigen.

Die in der Medizintechnik bei solchen Pumpen verwendeten Überleitsysteme werden üblicherweise nach jeder Behandlung ausgetauscht und nicht wieder für andere Patienten verwendet. Ein benutztes Schlauchsegment muss somit aus der Pumpe entfernt werden, bevor ein neues Überleitsystem in das Gerät eingebracht wird.

Ferner wird auch der Rotor eines solchen Systems üblicherweise nach jeder Behandlung zu Reinigungszwecken entnommen und nach der Reinigung wieder eingesetzt. Um die Handhabung des Rotors bei diesem Vorgang zu vereinfachen, sind bereits Systeme entwickelt worden, die zur Drehmomentübertragung von der Antriebswelle auf den Rotor einen radialen und axialen Formschluss verwenden. Derartig ausgestaltete Rotoren können insbesondere Bajonettverschlüsse in Kombination mit einem zusätzlichen Verriegelungselement aufweisen. Bei dem zusätzlichen Verriegelungselement kann es sich beispielsweise um einen Hebel handeln, der umgelegt werden muss, um den Rotor zu verriegeln. Jedoch sind für solche Systeme beim Einsetzvorgang wenigstens vier Handhabungsschritte erforderlich, denn es muss zunächst das zweite Verriegelungselement zur Drehkopplung umgelegt werden, bevor der Rotor auf die Antriebswelle aufgesteckt und um etwa 90° gedreht werden kann, um den Bajonettverschluss zu verrasten. Danach muss das Verriegelungselement wieder umgelegt werden, um den Rotor abschließend zu verriegeln.

Aufgabe der Erfindung ist es daher, eine Schlauchrollenpumpe für ein medizinisches Gerät zur extrakorporalen Blutbehandlung mit einem Rotor bereitzustellen, die ein Einsetzen des Rotors mit möglichst wenigen und leicht durchzuführenden Handhabungsschritte ermöglicht.

Aufgabe der Erfindung ist es ferner, ein medizinisches Gerät zur extrakorporalen Blutbehandlung bereitzustellen, in dessen Schlauchrollenpumpe ein solcher Rotor einbringbar ist.

Erfindungsgemäß wird diese Aufgabe durch eine Schlauchrollenpumpe gemäß dem unabhängigen Anspruch 1 gelöst. Vorteilhafte Weiterbildungen der Schlauchrollenpumpe ergeben sich aus den Unteransprüchen 2-12. Die Aufgabe wird ferner durch ein medizinisches Gerät zur extrakorporalen Blutbehandlung gemäß Anspruch 13 gelöst.

Die erfindungsgemäße Schlauchrollenpumpe für ein medizinisches Gerät zur extrakorporalen Blutbehandlung umfasst ein Pumpengehäuse, das eine gebogene Lauffläche und einen innerhalb der Lauffläche drehbaren Rotor aufweist, wobei ein Schlauchsegment zwischen die Lauffläche und den Rotor einbringbar ist. Dabei ist der Rotor mit einer Wellenaufnahme auf einer Antriebswelle der Schlauchrollenpumpe anbringbar, und es ist wenigstens ein Verriegelungselement zur axialen Sicherung und/oder Drehkopplung des Rotors auf der Antriebswelle vorgesehen.

Erfindungsgemäß sind am Rotor ein erstes Verriegelungselement zur wahlweisen axialen Sicherung des Rotors auf der Antriebswelle und ein zweites Verriegelungselement zur wahlweisen Drehkopplung von Antriebswelle und Rotor vorgesehen. Die Geometrien der Wellenaufnahme des Rotors und der Antriebswelle sind so ausgeformt, dass der Rotor beim axialen Aufschieben auf die Antriebswelle in eine erste vorbestimmte Drehlage gegenüber der Antriebswelle führbar ist, und das erste Verriegelungselement ist in dieser ersten Lage durch die Antriebswelle aus einer Verriegelungsposition in eine Entriegelungsposition bewegbar. Hierdurch ist der Rotor beim weiteren Aufschieben auf die Antriebswelle in eine zweite, axial definierte Lage bewegbar, in der sich das erste Verriegelungselement, insbesondere automatisch, zurück in die Verriegelungsposition bewegbar ist und das zweite Verriegelungselement, insbesondere manuell, so mit der Antriebswelle in Eingriff bringbar ist, dass ein Drehmoment von der Antriebswelle auf den Rotor übertragbar ist.

Durch diese Ausbildung des Rotors und der Antriebswelle der Pumpe ist der Rotor einerseits auto-orientierbar, da er automatisch in die richtige radial definierte Lage geführt wird, wenn der Bediener den Rotor axial auf die Antriebswelle aufschiebt. In dieser Lage kann der Rotor durch das zweite Verriegelungselement radial verriegelt werden, so dass ein Drehmoment von der Antriebswelle auf den Rotor übertragbar ist. Dabei wird der Rotor automatisch vom Benutzer in die richtige Position gedreht, wobei die Geometrie den Anwender dabei unterstützt, diese Position zu erreichen. Dies erleichtert dem Bediener den Einsetzvorgang erheblich.

Andererseits ist der Rotor auch auto-verriegelbar, da er beim Aufsetzvorgang automatisch durch das erste Verriegelungselement verriegelt wird, ohne dass der Bediener dazu beim Einsetzen einen weiteren Handgriff vornehmen muss. Vor dem Einsetzen befindet sich das Verriegelungselement dabei bereits in der Verriegelungsposition, und es wird nur temporär durch die Antriebswelle in die Entriegelungsposition bewegt, bevor es sich automatisch wieder zurückbewegt. Für das temporäre Entriegeln ist somit ebenfalls kein zusätzlicher Handgriff erforderlich, sondern die Antriebswelle entriegelt das Verriegelungselement beim Einsetzvorgang von selbst, und das erste Verriegelungselement ist so ausgestaltet, dass es automatisch wieder verriegelt, wenn der Rotor in einer zweiten, axial definierten Lage angekommen ist. In dieser Lage ist der Rotor auch in einer axial definierten Position, in welcher das zweite Verriegelungselement für eine Verdrehsicherung bzw. Drehkopplung mit der Antriebswelle in Eingriff gebracht werden kann.

In dieser Lage ist der Rotor somit einsatzbereit, und das erste Verriegelungselement kann den Rotor vorzugsweise nur in dieser Lage axial verriegeln. Die zuvor radial definierte Lage ermöglicht hierbei das axiale Verriegeln des Rotors und die Positionierung der Antriebswelle gegenüber dem zweiten Verriegelungs- bzw. Kraftübertragungselement.

Insgesamt ist es so möglich, eine axiale Sicherung und Verdrehsicherung einer lösbaren Welle-Nabe-Verbindung mit nur einem Handlingschritt herzustellen. Insbesondere ermöglicht das System eine Ein-Hand-Bedienung, da der Rotor vom Bediener mit einer Hand gegriffen und auf die Antriebswelle aufgeschoben werden kann. Bei demontiertem Rotor lässt sich auch die Antriebswelle gut reinigen.

In einem Ausführungsbeispiel der Erfindung ist das erste Verriegelungselement in der ersten, radial definierten Lage durch die Antriebswelle gegen eine Federkraft aus einer Verriegelungsposition in eine Entriegelungsposition bewegbar, während es in der zweiten axial definierten Lage durch die Federkraft zurück in die Verriegelungsposition bewegbar ist. Hierdurch kann auf einfache Weise eine automatische Entriegelung und erneute Verriegelung realisiert werden, die durch die axiale Bewegung des Rotors auf der Antriebswelle ausgelöst wird.

Die Antriebswelle und die Wellenaufnahme können jeweils Abgleitgeometrien aufweisen, die aneinander abgleiten, wodurch der Rotor beim axialen Aufschieben auf die Antriebswelle in die erste, radial definierte Lage gegenüber der Antriebswelle geführt wird. Vorzugsweise ist das erste Verriegelungselement dann in der ersten, radial definierten Lage durch die Abgleitgeometrie der Antriebswelle aus der Verriegelungsposition in die Entriegelungsposition bewegbar. Dies hat den Vorteil, dass die Abgleitgeometrie der Antriebswelle sowohl für die Drehausrichtung des Rotors in Bezug zur Antriebswelle, als auch für die Entriegelung des ersten Verriegelungselements genutzt werden kann.

Die Abgleitgeometrie an der Antriebswelle kann dabei beispielsweise durch einen giebelförmigen Endbereich ausgeformt sein, wodurch zwei schräge Flächen ausgebildet werden. Diese schrägen Flächen gleiten dann an der entsprechend ausgeformten Abgleitgeometrie der Wellenaufnahme ab, wodurch sich der Rotor in die gewünschte Drehlage dreht. Ferner drücken die schrägen Flächen das erste Verriegelungselement zur Seite, wobei das Verriegelungselement ebenfalls an einer schrägen Fläche abgleitet.

In einem Ausführungsbeispiel der Erfindung ist die Abgleitgeometrie der Wellenaufnahme durch zwei dreieckige Platten ausgeformt, die an der Innenwand der Wellenaufnahme ausgebildet sind und sich parallel gegenüber liegen, wobei eine jeweilige Spitze der dreieckigen Platten in Richtung des Rotorbodens zeigt. So kann der giebelförmige Endbereich der Antriebswelle an den dreieckigen Platten abgleiten und der Rotor wird dabei in eine definierte radiale Lage gedreht. Vorzugsweise weist die Antriebswelle dann unterhalb des giebelförmigen Endbereichs zwei gegenüber liegenden parallele Seitenflächen auf, und die parallelen Flächen der dreieckigen Platten innerhalb der Wellenaufnahme liegen in der ersten, radial definierten Lage des Rotors an den parallelen Seitenflächen der Antriebswelle an.

Das zweite Verriegelungselement weist vorzugsweise eine Nut auf und ist manuell in eine Position bringbar, in welcher gegenüber liegende Innenflächen dieser Nut an den Seitenflächen der Antriebswelle anliegen. Beispielsweise ist das zweite Verriegelungselement dazu um eine Schwenkachse innerhalb einer Aufnahme im Rotor schwenkbar. In einer ersten geöffneten Lage des radialen Verriegelungselements greift die Nut dann nicht an den Seitenflächen der Antriebswelle an, aber wenn das Verriegelungselement in Richtung der Antriebswelle umgelegt wird, umgreift die Nut von oben die Antriebswelle und liegt an den Seitenflächen der Antriebswelle an.

Das erste Verriegelungselement kann auf verschiedene Arten ausgebildet und auf die Geometrie der Antriebswelle abgestimmt sein, um diese axial verriegeln zu können. Das Verriegelungselement kann in der Verriegelungsposition beispielsweise in eine Rastgeometrie in der Antriebswelle eingreifen. In einem Ausführungsbeispiel der Erfindung handelt es sich bei dem ersten Verriegelungselement um ein quer zur Antriebswelle verlaufendes Blech bzw. Sicherungsblech, das in der Verriegelungsposition in eine radiale Nut der Antriebswelle eingreift. Hierbei ist die Nut vorzugsweise radial umlaufend ausgebildet, so dass der Rotor nach Erreichen der zweiten, axial definierten Lage noch um die Antriebswelle gedreht werden kann, wenn das zweite Verriegelungselement nicht verriegelt wird. Um das zweite Verriegelungselement zu verriegeln, müsste der Rotor dann nur wieder manuell in eine radiale Lage werden, in welcher ein Formschluss zwischen der Nut des radialen Verriegelungselements und den ebenen Seitenflächen der Antriebswelle herstellbar ist.

Das erste Verriegelungselement kann jedoch auch anders ausgeformt sein und beispielsweise einen oder mehrere Stifte umfassen. Auch Verriegelungen, die auf Reibung basieren, könnten eingesetzt werden, solange sie Bauteile umfassen, die beim Aufschieben des Rotors auf die Antriebswelle kontaktiert und bewegt werden können. Allerdings könnten auch Systeme eingesetzt werden, bei denen die Bewegung des Rotors auf der Antriebswelle auf andere Art die automatische Entriegelung bzw. Verriegelung bewirkt. Hierbei wären beispielsweise magnetische oder elektronische Systeme denkbar.

Um die axiale Verriegelung des Rotors manuell lösen zu können, bevor dieser beispielsweise nach der Therapie von der Antriebswelle abgezogen wird, kann der Rotor zusätzlich ein Betätigungselement aufweisen, mit dem das erste Verriegelungselement manuell aus der Verriegelungsposition in die Entriegelungsposition bewegbar ist. In einem Ausführungsbeispiel der Erfindung ist das manuelle Betätigungselement ein Hebel, der manuell umgelegt werden kann, um die Verriegelung zu lösen. Alternativ oder ergänzend kann als manuelles Betätigungselement auch ein Druckschalter eingesetzt werden.

Von der Erfindung umfasst ist ferner ein medizinisches Gerät zur extrakorporalen Blutbehandlung, das eine Schlauchrollenpumpe mit einem Pumpengehäuse aufweist, welches eine gebogene Lauffläche und einen innerhalb der Lauffläche drehbaren Rotor aufweist, wobei ein Schlauchsegment eines extrakorporalen Blutkreislaufs zwischen die Lauffläche und den Rotor einbringbar ist. Die Schlauchpumpe ist dabei gemäß einer Ausführungsform der erfindungsgemäßen Schlauchpumpe ausgeführt.

Weitere Vorteile, Besonderheiten und zweckmäßige Weiterbildungen der Erfindung ergeben sich aus den Unteransprüchen und der nachfolgenden Darstellung bevorzugter Ausführungsbeispiele anhand der Abbildungen.

Von den Abbildungen zeigt:
- Fig. 1: eine schematische Darstellung eines medizinischen Geräts zur extrakorporalen Blutbehandlung mit einer Blutpumpe;
- Fig. 2: eine schematische Aufsicht auf eine Schlauchrollenpumpe mit eingelegtem Schlauchstück und Rotor;
- Fig. 3a: eine schematische Seitenansicht eines Rotors am Anfang des Aufschiebens auf die Antriebswelle einer Schlauchrollenpumpe;
- Fig. 3b: eine schematische Aufsicht auf einen Rotor nach Figur 3a;
- Fig. 4a: eine schematische Seitenansicht eines Rotors gemäß Fig. 3a beim Entriegeln eines ersten Verriegelungselements;
- Fig. 4b: eine schematische Aufsicht auf einen Rotor nach Fig. 4a;
- Fig. 5a: eine schematische Seitenansicht eines Rotors gemäß Fig. 3a beim Verriegeln des ersten Verriegelungselements;
- Fig. 5b: eine schematische Aufsicht auf einen Rotor nach Fig. 5a;
- Fig. 6a: eine schematische Seitenansicht eines Rotor gemäß Fig. 3a mit verriegeltem zweiten Verriegelungselement; und
- Fig. 6b: eine schematische Aufsicht auf einen Rotor nach Fig. 6a.

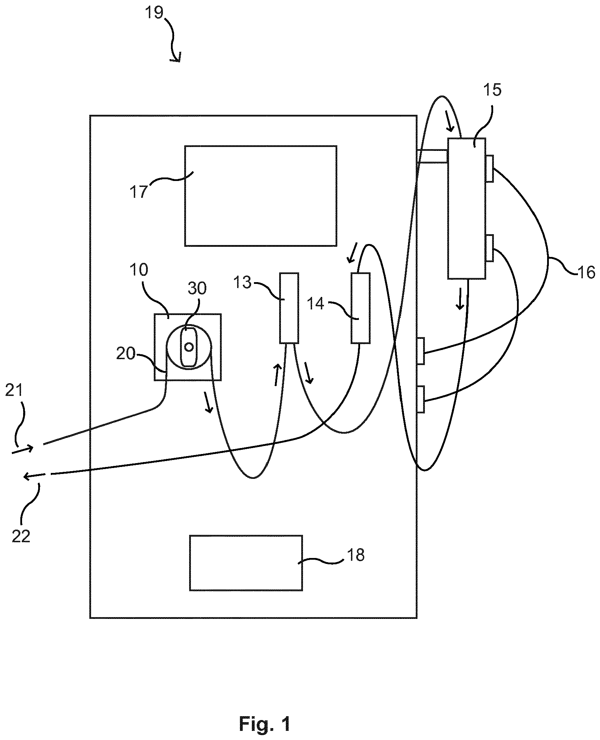

Fig. 1 zeigt eine schematische Darstellung der wesentlichen Grundkomponenten eines medizinischen Geräts 19 zur extrakorporalen Blutbehandlung mit einer Blutpumpe, wobei es sich bei der Blutpumpe um eine Schlauchrollenpumpe handelt. Die Schlauchrollenpumpe weist dabei ein Pumpengehäuse 10 auf, das typischerweise an der Frontseite des Dialysegeräts 19 angebracht ist.

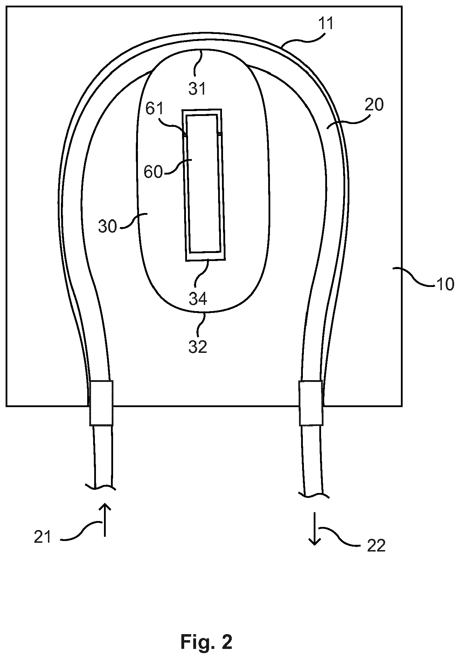

Dieser Schlauchrollenpumpe wird arterielles Blut 21 eines Patienten zugeführt und durch den extrakorporalen Blutkreislauf gefördert. Anschließend wird das Blut als venöses Blut 22 wieder zum Patienten zurückgeführt. Dabei wird das Blut mittels der Pumpe durch ein Überleitsystem gefördert, das an mehrere Komponenten des Dialysegeräts angeschlossen ist, wobei ein Schlauchsegment 20 des Überleitsystems in die Blutpumpe eingelegt ist und ein Rotor 30 das Blut peristaltisch durch dieses Schlauchsegment 20 fördert, wie es einer vergrößerten Ansicht der Fig. 2 zu entnehmen ist.

Nach Durchlaufen der Blutpumpe gelangt das Blut zum Dialysator 15, nachdem es vorzugsweise zuvor einen arteriellen Luftfänger 13 durchlaufen hat. Im Dialysator 15 wird das Blut durch Stoffaustausch mit einem Dialysat 16 gereinigt, welches dem Dialysator 15 zu- und abgeführt wird. Nach Durchlaufen des Dialysators 15 gelangt das Blut zu einem venösen Luftfänger 14 und wird anschließend dem Patienten zugeführt. Dieser Kreislauf des Bluts des Patienten ist in Fig. 1 durch Pfeile gekennzeichnet.

Die Einstellung von Parametern der Dialyse und die Überwachung der Therapie können über eine Anzeige-/Eingabeeinheit 17 erfolgen, die vorzugsweise als Touchscreen ausgebildet ist. Ferner weist das Dialysegerät 19 eine Steuer-/Regeleinheit 18 auf.

Fig. 2 stellt eine schematische Darstellung einer Aufsicht auf eine Schlauchrollenpumpe mit eingelegtem Schlauchsegment 20 und Rotor 30 dar. Die Schlauchrollenpumpe ist für den Bediener des Geräts leicht zugänglich, wobei das Pumpengehäuse 10 mit einem nicht dargestellten Deckel abdeckbar ist, der über ein Scharnier zum Beispiel nach oben oder zur Seite schwenkbar ist, um Zugriff auf das Schlauchsegment 20 zu erhalten.

In dem Pumpengehäuse 10 ist durch eine Vertiefung im Gehäuse eine kurvenförmige Lauffläche 11 ausgebildet, in welche das Schlauchsegment 20 schlaufenförmig eingelegt werden kann, so dass seine beiden Schlauchenden unten aus dem Gehäuse 10 herausragen. Dabei kann die Vertiefung mit einer Seitenfläche in dem Pumpengehäuse 10 ausgebildet sein, die im Wesentlichen gleichmäßig senkrecht zur Frontseite des Geräts verläuft, oder die Lauffläche 11 ist ungleichmäßig durch eine Seitenfläche der Vertiefung ausgeformt, die konkav oder sogar in sich verdreht ausgeformt ist.

Innerhalb der Lauffläche 11 ist ein Rotor 30 angebracht, der beispielsweise einen in etwa elliptischen Umfang hat, so dass er das Schlauchsegment 20 bei der Rotation an seinen Hauptscheiteln 31, 32 mit nicht dargestellten Rollen leicht zusammendrücken kann. Durch die Drehung des Rotors 30 im Uhrzeigersinn bewegt sich der Bereich eines zusammengedrückten Schlauchsegments ebenfalls im Uhrzeigersinn, bis sich der zugehörige Hauptscheitel 31 bzw. die daran angebrachte Rolle wieder vom Schlauchsegment löst. In der Zeit hat der gegenüber liegende Hauptscheitel 32 jedoch bereits wieder Kontakt zu dem Schlauchsegment 20 aufgenommen, so dass Blut jeweils in dem Bereich des Schlauchsegments 20, vor dem es von dem Rotor 30 zusammendrückt wird, peristaltisch vom Pumpeneingang zum Pumpenausgang gefördert wird.

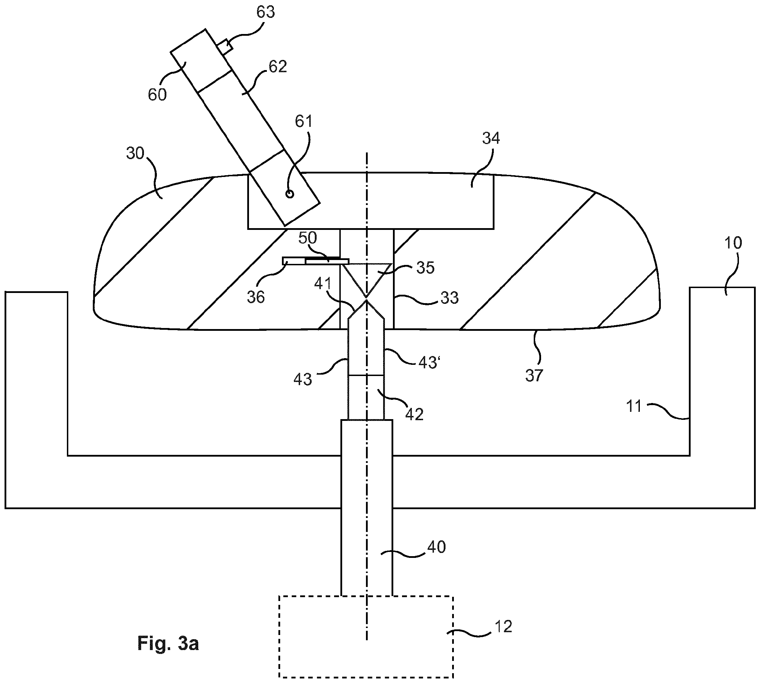

Fig. 3a zeigt eine schematische Darstellung eines Rotors 30 am Anfang der Montage auf einer Antriebswelle 40 einer Schlauchrollenpumpe. Die Antriebswelle 40 befindet sich innerhalb des Pumpengehäuses 10, in dem die Lauffläche 11 zur Aufnahme des Schlauchsegments ausgebildet ist. Die Antriebswelle 40 der Pumpe ist gleichzeitig die Abtriebswelle bzw. Getriebeabtriebswelle eines Antriebs 12, der in Fig. 3a lediglich gestrichelt dargestellt ist.

In der Mitte des Rotors 30 ist eine Wellenaufnahme 33 vorgesehen, um den Rotor 30 auf die Antriebswelle 40 aufschieben zu können. Die Wellenaufnahme 33 ist im Wesentlichen hohlzylindrisch ausgeführt, weist jedoch bereichsweise eine andere Geometrie auf, beispielsweise mit dreieckigen Plattenflächen 35, 35' an der Innenfläche der Wellenaufnahme 33. Um das Drehmoment von der Antriebswelle 40 auf den Rotor 30 zu übertragen, ist im oberen Bereich des Rotors 30 bzw. vom Antrieb 12 abgewandten Seite des Rotors 30 ein (zweites) Verriegelungselement bzw. Verdrehsicherungselement bzw. Kraftübertragungselement 60 angebracht. Dieses Verdrehsicherungselement 60 ist dabei innerhalb einer Aufnahme 34 im Rotor 30 um eine Achse 61 schwenkbar gelagert, um zwischen einer Ent- und Verriegelungsstellung hin- und her geschwenkt werden zu können. Das Verdrehsicherungselement 60 weist ferner eine Nut 62, in welche in der Verriegelungsstellung ein Endabschnitt der Antriebswelle 40 formschlüssig eingreift.. Ferner kann am Verdrehsicherungselement 60 ein Kurbelgriff 63 für den manuellen Notbetrieb vorgesehen sein. In der Situation der Fig. 3a vor dem Aufsetzen des Rotors 30 auf die Antriebswelle ist das Verdrehsicherungselement 60 nach links bzw. nach außen geschwenkt und entriegelt. Das radiale Verdrehsicherungselement 60 muss jedoch nicht geöffnet sein, um den Rotor 30 in die Therapieposition bzw. Arbeitsstellung aufzusetzen, da der Rotor 30 durch die Geometrien der Antriebswelle 40 und der Wellenaufnahme 33 automatisch in eine bestimmte relative Drehlage geführt wird, in welcher das obere Ende der Antriebswelle 40 formschlüssig in die Nut 62 des radialen Verriegelungselements 60 eingreift.

Im Bereich der Wellenaufnahme 33 ist ferner ein (erstes) Verriegelungselement 50 zur axialen Festlegung des Rotors 30 auf der Antriebswelle 40 angeordnet, das in der Verriegelungsposition radial nach innen in die Wellenaufnahme 33 hineinragt. Dieses Verriegelungselement 50 ist radial beweglich innerhalb einer Aussparung 36 im Rotor 30 angebracht, wobei es vorzugsweise federbelastet ist und durch Federkraft (einer nicht dargestellten Feder) radial nach innen vorgespannt ist und zur Wellenaufnahme 33 hin gedrückt wird. Innerhalb der Antriebswelle 40 ist beispielsweise eine in Umfangsrichtung umlaufende Nut 42 vorgesehen, in welche das Verriegelungselement 50 in der Verriegelungsposition eingreift, so dass der Rotor 30 axial an der Antriebswelle 40 verriegelt ist. Die Verriegelung kann jedoch auch durch jegliche andere Geometrien der Antriebswelle 40 erreicht werden, in welche das Verriegelungselement 50 einrasten kann.

Beim Einsetzen des Rotors 30 in die Pumpe kann dieser von einem Bediener gegriffen und mit entriegeltem bzw. weggeschwenkten Verdrehsicherungselement 60 auf die Antriebswelle 40 aufgeschoben werden. Die Geometrien der Antriebswelle 40 und der Wellenaufnahme 33 sind dabei so ausgebildet und aufeinander abgestimmt, dass der Rotor 30 dabei automatisch in eine erste, definierte relative Drehlage gegenüber der Antriebswelle 40 geführt wird. Dazu ist der dem Rotor 30 zugewandte Endabschnitt 41 der Antriebswelle 40 keilförmig ausgebildet, so dass sich zwei schräge Keilflächen bilden, die in Fig. 3a nach links und rechts abfallen. Diese schrägen Flächen gehen in zwei gegenüber liegende, zur Drehachse der Antriebswelle 40 parallele ebene Seitenflächen bzw. Abflachungen 43 und 43' an der Antriebswelle 40 über.

Gleichzeitig sind innerhalb der Wellenaufnahme 33 an der Innenwandung zwei dreieckige Platten 35 und 35' mit zur Drehachse des Rotors 30 parallele Innenflächen ausgeformt, von denen in der Darstellung der Fig. 3a nur die hintere Platte 35 dargestellt ist. Eine zweite dreieckige Platte 35' befindet sich parallel zur ersten Platte 35 auf der gegenüber liegenden Seite der Wellenaufnahme 33, die in der Ansicht der Fig. 3a weggeschnitten ist. Die Platten 35 und 35' bilden ein gleichschenkliges Dreieck und sind dabei so ausgerichtet, dass deren Spitze jeweils in Richtung des Rotorbodens 37 zeigt.

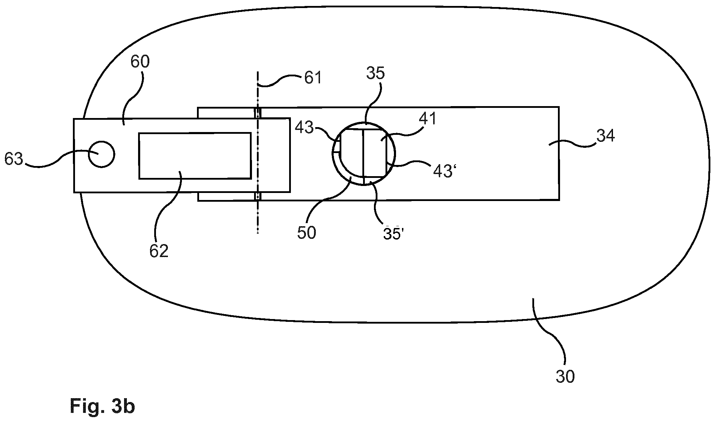

In der Situation der Fig. 3a wurde der Rotor 30 von einem Benutzer so auf der Antriebswelle 40 positioniert, dass die Spitze des keilförmigen Endabschnitts der Antriebswelle 40 in etwa auf die beiden Spitzen der dreieckigen Platten 35 und 35' trifft. Dies ist auch aus der Aufsicht der Fig. 3b ersichtlich, welche innerhalb der Wellenaufnahme 33 die beiden gegenüber liegenden dreieckigen Platten 35 und 35' zeigt. Das Verriegelungselement 50 befindet sich (noch) oberhalb der Platten 35, 35', und die Oberkante der keilförmigen Stirn der Antriebswelle 40 ist quer zu den Innenflächen der Platten 35, 35' ausgerichtet. Ferner sind in dieser Ansicht die ebenen Seitenflächen 43 und 43' der Antriebswelle 40 gezeigt, die ebenfalls quer zu den Platten 35, 35'ausgerichtet sind.

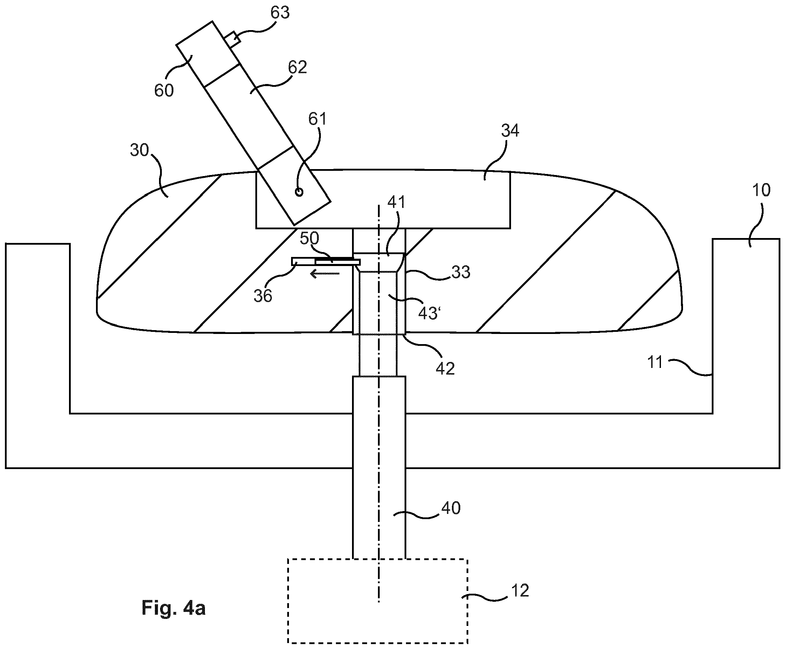

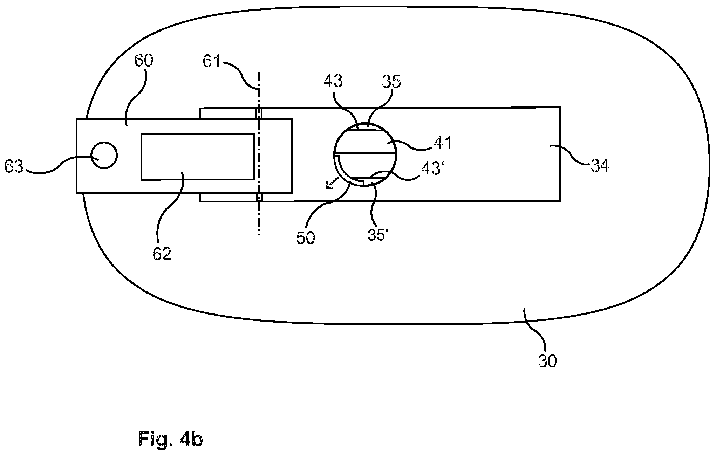

Da in dieser Lage die äußeren Abschnitte des keilförmigen Endabschnitts 41 der Antriebswelle 40 auf die Spitze der Platte 35 bzw. Platte 35' trifft, kann der Rotor 30 nicht auf die Antriebswelle 40 aufgesetzt werden. Aber bei leichtem Drehen und Druck gleitet der keilförmige Endabschnitt 41 der Antriebswelle 40 mit seinen Keilflächen an den Flanken bzw. Schenkeln der dreieckigen Platten 35, 35' ab, wobei der Rotor 30 in eine Drehung gezwungen wird, welche der Benutzer registriert und der er mit seiner Hand folgen kann. Wenn die Rotor 30 und die Antriebswelle 40 von der in der Fig. 3a und 3b gezeigten Ausrichtung relative zueinander um 90° gedreht wurden, so nehmen sie die in den Fig. 4a und 4B gezeigten Drehlage bzw. Ausrichtung ein, in der die ebenen Seitenflächen 43 und 43' der Antriebswelle 40 nun nach vorne zeigen. Sie sind somit parallel zu den Innenflächen der dreieckigen Platten 35 und 36 (in Fig. 4a hinter der Antriebswelle) ausgerichtet. Ferner fallen nun die Keilflächen des Endabschnitts 41 der Antriebswelle 40 nach vorne und hinten ab.

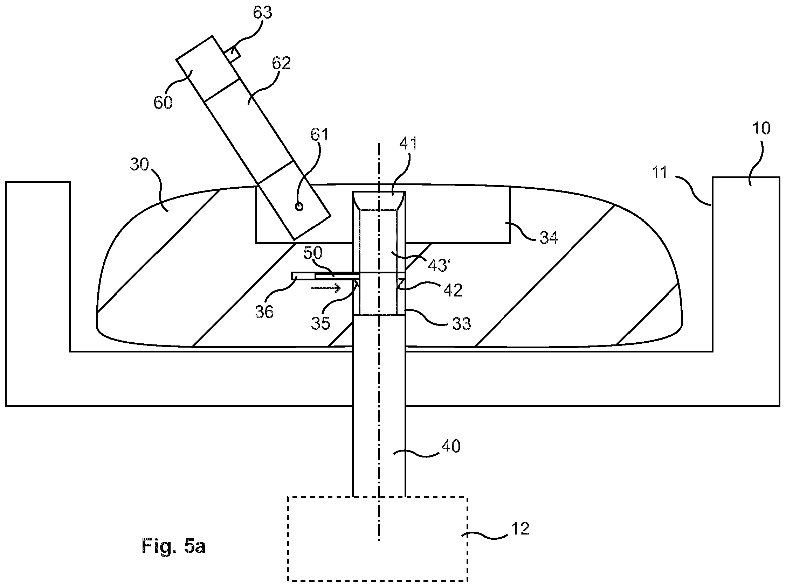

Dies ist auch aus der Aufsicht der Fig. 4b ersichtlich, wobei die Seitenflächen 43 und 43' nun an den Innenflächen der Platten 35 und 35' anliegen. Dabei sind die Platten 35, 35' und somit auch die Seitenflächen 43, 43' der Antriebswelle 40 vorzugsweise parallel zur Längsachse des Rotors 30 ausgerichtet. In der in den Fig. 4a und 4b gezeigten Lage befinden sich der Rotor 30 und die Antriebswelle zueinander in einer ersten, definierten Drehlage, aber ein weiteres Aufschieben des Rotors 30 auf die Antriebswelle 40 ist eigentlich durch das Verriegelungselement 50 blockiert. Allerdings befinden sich nun der Rotor 30 und die schrägen Keilflächen im Endbereich der Antriebswelle 40 in einer definiertenAusrichtung, in der das Verriegelungselement 50 eine der Keilflächen kontaktiert und diese Keilfläche gegen eine Federkraft des radial nach innen vorgespannten Verriegelungselements 50 radial nach außen in die Aussparung 36 verdrängen kann, wenn der Rotor 30 in axialer Richtung weiter geschoben wird. Diese Bewegung des Verriegelungselements 50 ist in den Figuren 4a und 4b durch einen Pfeil nach links bzw. nach links unten dargestellt. Dabei gleitet das Verriegelungselement 50 an dem keilförmigen Endabschnitt 41 der Antriebswelle 40 ab, wodurch der Rotor 30 weiter auf die Antriebswelle 40 geschoben werden kann, bis er schließlich in eine zweite, axial definierte Lage gelangt, die in Fig. 5a dargestellt ist. Dabei ist zu beachten, dass die Aussparung 36, in der das Verriegelungselement 50 radial geführt ist, wie insbesondere in der Fig. 4 gezeigt ist, in der definierten ersten relativen Drehlage der Antriebswelle 40 so angeordnet ist, dass es sich seitlich zur Kante des keilförmigen Endabschnitt 41 befindet, dass Verriegelungselement 50 lediglich mit einer der Keilflächen und nicht mit der Keilkante in Kontakt kommt und sichergestellt werden kann, dass Verriegelungselement 50 an den Keilflächen abgleiten kann. Dies kann erreicht werden, indem wie in der Fig. 4 gezeigt ist, die Aussparung 36 und das Verriegelungselement 50 mehr auf der Seite einer der Dreiecksplatten 35' befindet, welche die Ausrichtung der Antriebswelle 40 und somit der Keilflächen definieren.

Ferner sollte auch die radiale Bewegung des Verriegelungselements 50 nach innen, z.B. durch einen (nicht gezeigten) Anschlag, begrenzt sein, damit das Verriegelungselement 50 nicht in Überdeckung mit der Keilkante gelangt.

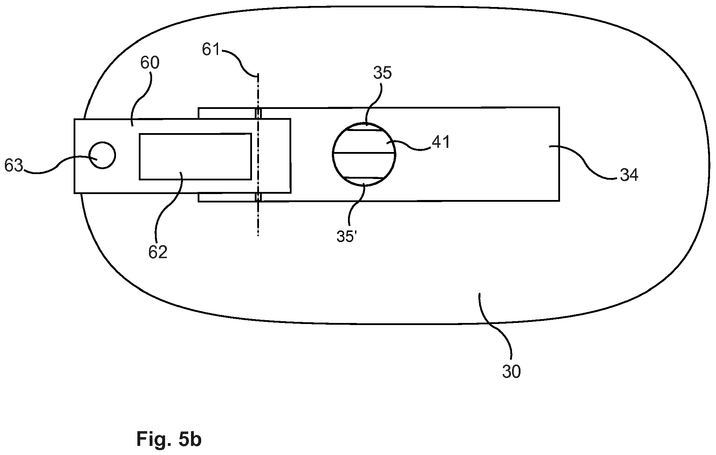

In der in Fig. 5a gezeigten, axial definierten Lage befindet sich die Nut 42 der Antriebswelle 40 im Bereich des Verriegelungselements 50, so dass sich dieses durch die Federkraft in die Nut 42 bewegt und somit in diese eingreift. Diese Bewegung des Verriegelungselements 50 ist in Fig. 5a durch einen Pfeil nach rechts dargestellt. Das Verriegelungselement 50 liegt dann innen bzw. von untenan einer Seitenwand der Nut 42 an und ist in der Aufsicht der Fig. 5b nicht mehr zu sehen. Die Breite der Nut 42 und die axiale Höhe der dreieckigen Platten 35 und 35' sind so gewählt, dass die Spitzen der Platten 35 und 35' in dieser Lage in Anlage mit der anderen (unteren) Seitenwand der Nut 42 kommen, so dass der Rotor 30 einerseits nicht mehr von der Antriebswelle gezogen and andererseits nicht mehr weiter auf die Antriebswelle 40 aufgeschoben werden kann. Hierdurch wird der Rotor 30 gegen axiale Bewegungen auf der Antriebswelle 40 gesichert. Alternativ kann die Breite der Nut 42 und die Dicke des Verriegelungselements 50 so aufeinander abgestimmt sein, dass das Verriegelungselement 50 im Zusammenwirken mit der Nut 42 eine axiale Relativbewegung von Rotor 30 und Antriebswelle 40 alleine verhindert.

Handelt es sich um eine radial umlaufende Nut 42, kann der Rotor 30 zwar nicht mehr von der Antriebswelle 40 abgezogen werden, aber er kann auf der Antriebswelle 40 solange gedreht werden, bis das radiale Verdrehsicherungselement 60 betätigt wird. In weg- bzw. zur Seite geschwenkten Lage des Verdrehsicherungselements 60 ist somit zwar eine axiale Verriegelung gegeben, aber der Rotor 30 kann beispielsweise für einen manuellen Notbetrieb an dem Kurbelgriff 63 gedreht werden. So kann im Notbetrieb auf manuelle Weise dem Patienten sein Blut wieder aus dem Leitungssystem zugeführt werden, ohne dass man beim Kurbeln Gefahr läuft, dass der Rotor 30 von der Antriebswelle 40 rutscht.

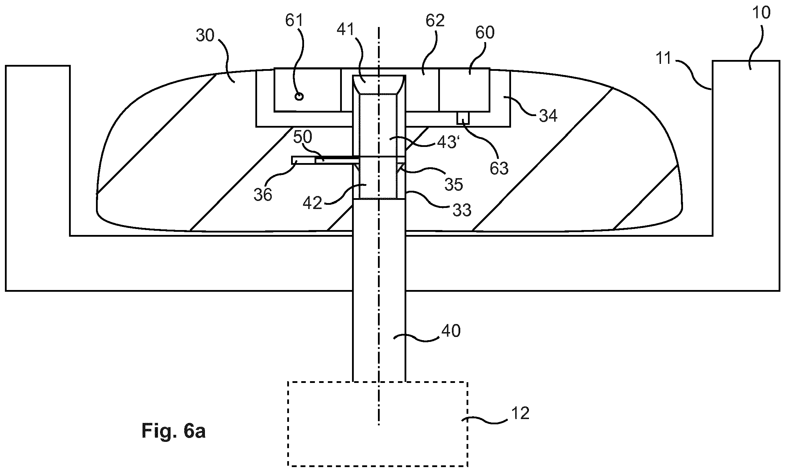

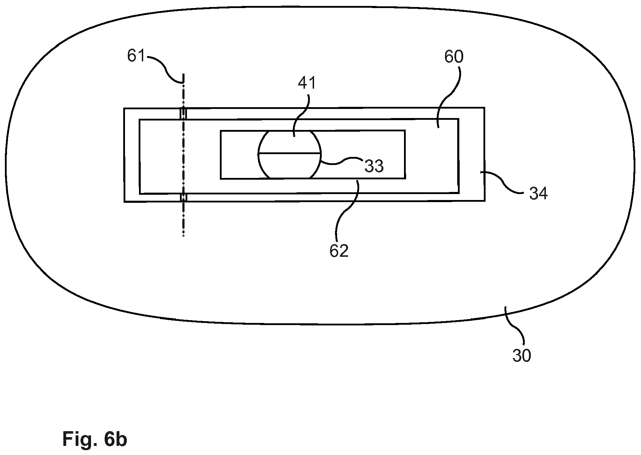

Um den Rotor 30 für eine Therapie bzw. in der Arbeitsstellung auch radial zu verriegeln, wird das Verdrehsicherungselement 60 um die zur Drehachse des Rotors senkrecht verlaufende und zu dieser beabstandete Achse 61 auf die Antriebswelle 40 geschwenkt, wie es in der Situation der Fig. 6a gezeigt ist. Die Innenflächen der Nut 62 innerhalb des Verriegelungselements 60 liegen dann an den Seitenflächen 43 und 43' der Antriebswelle 40 flächig an, so dass aufgrund des Formschlusses keine Relativdrehung zwischen Antriebswelle 40 und Rotor 30 mehr möglich ist und ein Drehmoment von der Antriebswelle 40 auf den Rotor 30 übertragbar ist. Aus der Fig. 6a ist ferner zu erkennen, dass der Kurbelgriff 63 zugleich als Anschlag dienen kann, um die Schwenkbewegung des Verdrehsicherungselements 60 in der Verriegelungsstellung zu begrenzen.

Zur Demontage des Rotors 30 nach einer Therapie könnte das Verdrehsicherungselement 60 wieder weg (in der Fig. 6a nach links) geschwenkt und so entriegelt werden. Dies ist jedoch nicht zwingend erforderlich, sondern die Verdrehsicherung kann auch durch einfaches Abziehen des Rotors 30 von der Antriebswelle 40 gelöst werden. Dazu muss das axiale Verriegelungselement 50 gelöst werden, um den Rotor 30 von der Antriebswelle 40 ziehen zu können. Hierzu kann am Rotor 30 ein manuelles Betätigungselement vorgesehen sein, wobei es sich beispielsweise um einen Hebel handeln kann, der umgelegt wird, um das Verriegelungselement 50 gegen die Federkraft aus der Verriegelungsposition radial nach außen in die Entriegelungsposition zu bewegen. Nach der Abnahme des Rotors 30 kann dieser Hebel dann wieder umgelegt werden, so dass sich das Verriegelungselement 50 vor dem erneuten Aufsetzen auf die Antriebswelle 40 wieder in der Verriegelungsposition befindet, um die beschriebene Verbindung mit nur einem Handhabungsschritt realisieren zu können. Es kann jedoch auch vorgesehen sein, dass der Hebel wie oben bereits beschrieben durch Federkraft automatisch wieder in die Position zurückkehrt, in welcher sich das Verriegelungselement 50 in der Verriegelungsposition befindet.

Das manuelle Betätigungselement kann jedoch beispielsweise auch ein Druckschalter am Rotor 30 sein, der von einem Bediener niedergedrückt werden muss, um den Rotor 30 abzuziehen. Vorzugsweise ist der Schalter dabei so positioniert, dass er beim Ergreifen des Rotors 30 gleichzeitig niedergedrückt werden kann. Dabei bewirkt ein Niederdrücken des Schalters eine Bewegung des Verriegelungselements 50 in die Entriegelungsposition, aber das Verriegelungselement 50 kehrt automatisch in die Verriegelungsposition zurück, wenn der Druckschalter wieder losgelassen wird. Dies kann erneut über Federkraft erfolgen. Alternativ kann ein weiterer Druckschalter vorgesehen sein, um eine Bewegung des Verriegelungselements 50 von der Entriegelungsposition in die Verriegelungsposition zu bewirken.

Ferner kann das radiale Verdrehsicherungselement 60 auch anders ausgebildet werden als durch ein schwenkbares Bauteil. Beispielsweise kann es sich um einen Einsatz, z.B. einen Schieber, mit einem Griffelement handeln, der von einem Benutzer innerhalb der Aussparung 34 im Rotor 30 hin- und herbewegt werden kann. Innerhalb des Einsatzes ist dann eine Nut ausgeformt, welche bei richtiger Ausrichtung des Einsatzes bezüglich des Rotors und somit der Antriebswelle den Formschluss mit der Antriebswelle 40 herstellt, wenn der Einsatz in den Rotor 30 hineingedrückt wird oder auf den Rotor 30 geschoben wird. Zum Entriegeln muss der Einsatz dann wieder leicht aus dem Rotor 30 heraus- bzw. weggezogen werden.

Gegenüber dieser Ausführungsform eines Verdrehsicherungselements hat die zuvor beschriebene Variante jedoch den Vorteil, dass ein Kurbelgriff 63 für den manuellen Notbetrieb besser in das Verriegelungselement 60 integrierbar ist, da der Kurbelgriff 63 bei einem schwenkbaren Verdrehsicherungselement 60 aus der Drehachse des Rotors 30 heraus in Richtung des Rands des Rotors 30 bewegt wird.

### Bezugszeichenliste

10 Pumpengehäuse
11 Lauffläche
12 Antrieb
13 Arterieller Luftfänger
14 Venöser Luftfänger
15 Dialysator
16 Dialysat
17 Anzeige-/Eingabeeinheit, Touchscreen
18 Steuer-/Regelungseinheit
19 Medizinisches Gerät zur extrakorporalen Blutbehandlung, Dialysegerät
20 Schlauchsegment
21 Arterielles Blut vom Patienten
22 Venöses Blut zum Patienten
30 Rotor
31, 32 Hauptscheitel
33 Wellenaufnahme
34 Aufnahme
35, 35' Abgleitgeometrie, dreieckige Platte
36 Aussparung
37 Rotorboden
40 Antriebswelle, Abtriebswelle der Pumpe
41 Abgleitgeometrie, keilförmig
42 Nut, Rastgeometrie
43, 43' Seitenfläche der Antriebswelle
50 Erstes Verriegelungselement, Sicherungsblech
60 Zweites Verriegelungselement, Verdrehsicherungselement, Kraftübertragungselement
61 Schwenkachse des Verdrehsicherungselements
62 Nut im Verdrehsicherungselement
63 Kurbelgriff

## Patentansprüche

1. Schlauchrollenpumpe für ein medizinisches Gerät zur extrakorporalen Blutbehandlung mit einem Pumpengehäuse (10), das eine gebogene Lauffläche (11) und einen innerhalb der Lauffläche (11) drehbaren Rotor (30) aufweist, wobei ein Schlauchsegment (20) zwischen die Lauffläche (11) und den Rotor (30) einbringbar ist, und der Rotor (30) mit einer Wellenaufnahme (33) auf einer Antriebswelle (40) der Schlauchrollenpumpe anbringbar ist, wobei wenigstens ein Verriegelungselement (50;60) zur axialen Sicherung und/oder Drehkopplung des Rotors (30) auf der Antriebswelle (40) vorgesehen ist,
wobei
am Rotor (30) ein erstes Verriegelungselement (50) zur wahlweise axialen Sicherung des Rotors (30) auf der Antriebswelle (40) und ein zweites Verriegelungselement (60) zur wahlweisen Drehkopplung von Antriebswelle (40) und Rotor (30) vorgesehen sind,
**dadurch gekennzeichnet, dass**
die Geometrien der Wellenaufnahme (33) des Rotors (30) und der Antriebswelle (40) so ausgeformt sind, dass der Rotor (30) beim axialen Aufschieben auf die Antriebswelle (40) in eine erste vorbestimmte relative Drehlage gegenüber der Antriebswelle (40) führbar ist, und das erste, bewegliche Verriegelungselement (50) in dieser ersten Lage durch die Antriebswelle (40) aus einer Verriegelungsposition in eine Entriegelungsposition bewegbar ist, wodurch der Rotor (30) beim weiteren Aufschieben auf die Antriebswelle (40) in eine zweite, axial definierte Lage bewegbar ist, in der das erste Verriegelungselement (50), insbesondere automatisch, zurück in die Verriegelungsposition bewegbar ist und das zweite Verriegelungselement (60), insbesondere manuell, so mit der Antriebswelle (40) in Eingriff bringbar ist, dass ein Drehmoment von der Antriebswelle (40) auf den Rotor (30) übertragbar ist

2. Schlauchrollenpumpe nach Anspruch 1,
**dadurch gekennzeichnet, dass** das erste Verriegelungselement (50) in der ersten Lage durch die Antriebswelle (40) gegen eine Federkraft aus einer Verriegelungsposition in eine Entriegelungsposition bewegbar ist, während das axiale Verriegelungselement (50) in der zweiten Lage durch die Federkraft zurück in die Verriegelungsposition bewegbar ist.

3. Schlauchrollenpumpe nach einem oder beiden der Ansprüche 1 und 2,
**dadurch gekennzeichnet, dass** die Antriebswelle (40) und die Wellenaufnahme (33) jeweils Abgleitgeometrien (41, 35, 35') aufweisen, die aneinander abgleiten, wenn der Rotor (30) beim axialen Aufschieben auf die Antriebswelle (40) in die erste Lage gegenüber der Antriebswelle (40) geführt wird.

4. Schlauchrollenpumpe nach Anspruch 3,
**dadurch gekennzeichnet, dass** das erste Verriegelungselement (50) in der ersten Lage durch Kontakt mit der Abgleitgeometrie (41) der Antriebswelle (40) aus der Verriegelungsposition in die Entriegelungsposition bewegbar ist.

5. Schlauchrollenpumpe nach einem oder beiden der Ansprüche 3 und 4,
**dadurch gekennzeichnet, dass** die Abgleitgeometrie (41) an der Antriebswelle (40) durch einen keilförmigen Endabschnitt ausgeformt ist, wodurch zwei zur Drehachse der Antriebswelle (40) angestellte Keilflächen ausgebildet werden.

6. Schlauchrollenpumpe nach einem oder mehreren der Ansprüche 3 bis 5,
**dadurch gekennzeichnet, dass** die Abgleitgeometrie der Wellenaufnahme (33) durch zwei dreieckige Platten (35;35') ausgeformt ist, die an der Innenwand der Wellenaufnahme (33) ausgebildet sind, zur Drehachse des Rotors (30) parallele Innenflächen aufweisen und einander gegenüber liegen, wobei eine Spitze der dreieckigen Platten (35, 35') jeweils in Richtung des Rotorbodens (37) zeigt.

7. Schlauchrollenpumpe nach Anspruch 6,
**dadurch gekennzeichnet, dass** die Innenflächen der dreieckigen Platten (35, 35') innerhalb der Wellenaufnahme (33) in der ersten Lage des Rotors (30) an zueinander planparallelen Seitenflächen (43, 43'), die an zwei gegenüber liegende Seiten der Antriebswelle (40) ausgebildet sind, flächig anliegen.

8. Schlauchrollenpumpe nach Anspruch 7,
**dadurch gekennzeichnet, dass** das zweite Verriegelungselement (60) eine Nut (62) aufweist und manuell in eine Position bringbar ist, in welcher einander gegenüber liegende Innenflächen dieser Nut (62) an den Seitenflächen (43, 43') der Antriebswelle (40) flächig anliegen.

9. Schlauchrollenpumpe nach einem oder mehreren der Ansprüche 1 bis 8,
**dadurch gekennzeichnet, dass** das zweite Verriegelungselement (60) um eine, insbesondere senkrecht zur Drehachse des Rotors (30) und zu dieser beabstandete, Schwenkachse (61) innerhalb einer Aufnahme (34) im Rotor (30) schwenkbar ist.

10. Schlauchrollenpumpe nach einem oder mehreren der Ansprüche 1 bis 9 **dadurch gekennzeichnet, dass** das erste Verriegelungselement (50) in der Verriegelungsposition in eine Rastgeometrie (42) in der Antriebswelle (40) eingreift.

11. Schlauchrollenpumpe nach einem oder mehreren der Ansprüche 1 bis 10,
**dadurch gekennzeichnet, dass** das erste Verriegelungselement (50) ein radial zur Drehachse des Rotors bewegbares flaches Blechteil ist, das in der Verriegelungsposition in eine in Umfangsrichtung umlaufende Nut (42) der Antriebswelle (40) eingreift.

12. Schlauchrollenpumpe nach einem oder mehreren der Ansprüche 1 bis 11,
**dadurch gekennzeichnet, dass** der Rotor (30) ein Betätigungselement, insbesondere einen Hebel oder einen Druckschalter, aufweist, mit dem das erste Verriegelungselement (50) manuell aus der Verriegelungsposition in die Entriegelungsposition bewegbar ist.

13. Medizinisches Gerät zur extrakorporalen Blutbehandlung, umfassend eine Schlauchrollenpumpe mit einem Pumpengehäuse (10), das eine gebogene Lauffläche (11) und einen innerhalb der Lauffläche (11) drehbaren Rotor (30) aufweist, wobei ein Schlauchsegment (20) eines extrakorporalen Blutkreislaufs zwischen die Lauffläche (11) und den Rotor (30) einbringbar ist,
**dadurch gekennzeichnet, dass** die Schlauchrollenpumpe nach einem oder mehreren der Ansprüche 1 bis 12 ausgebildet ist.

## Claims

1. A tube roller pump for a medical device for extracorporeal blood treatment, comprising a pump housing (10) including a curved running surface (11) and a rotor (30) that is rotatable within the running surface (11), wherein a tube segment (20) is adapted to be placed between the running surface (11) and the rotor (30), and the rotor (30) is adapted to be attached via a shaft reception means (33) to a drive shaft (40) of the tube roller pump, and wherein at least one locking element (50; 60) is provided for axially locking and/or rotationally coupling the rotor (30) to the drive shaft (40),
wherein
the rotor (30) has provided thereon a first locking element (50) for selective axial locking of the rotor (30) on the drive shaft (40) and a second locking element (60) for selective rotational coupling of drive shaft (40) and rotor (30),
**characterized in that**
the geometry of the shaft reception means (33) of the rotor (30) and that of the drive shaft (40) are configured such that the rotor (30) is adapted to be guided to a first predetermined rotary position relative to the drive shaft (40), when it is axially pushed onto the drive shaft (40), and that, at this first position, the first, movable locking element (50) is adapted to be moved by the drive shaft (40) from a locking position to a release position, whereby the rotor (30), when it is pushed further onto the drive shaft (40), is adapted to be moved to a second, axially defined position at which the first locking element (50) is adapted to be moved back to the locking position, in particular automatically, and the second locking element (60) is adapted to be brought, in particular manually, into engagement with the drive shaft (40) such that a torque can be transmitted from the drive shaft (40) to the rotor (30).

2. The tube roller pump according to claim 1,
**characterized in that,** at the first position, the first locking element (50) is adapted to be moved by means of the drive shaft (40) from a locking position to a release position against the force of a spring, whereas, at the second position, the axial locking element (50) is adapted to be moved back to the locking position by the force of said spring.

3. The tube roller pump according to one or both of claims 1 and 2,
**characterized in that** the drive shaft (40) and the shaft reception means (33) both have slide geometries (41, 35, 35') sliding along one another, when the rotor (30), while being axially pushed onto the drive shaft (40), is guided to the first position relative to the drive shaft (40).

4. The tube roller pump according to claim 3,
**characterized in that,** at the first position, the first locking element (50) is adapted to be moved from the locking position to the release position through contact with the slide geometry (41) of the drive shaft (40).

5. The tube roller pump according to one or both of claims 3 and 4,
**characterized in that** the slide geometry (41) on the drive shaft (40) is defined by a wedge-shaped end portion, whereby two wedge surfaces inclined at an angle relative to the axis of rotation of the drive shaft (40) are formed.

6. The tube roller pump according to one or more of claims 3 to 5,
**characterized in that** the slide geometry of the shaft reception means (33) is defined by two triangular plates (35, 35'), which are formed on the inner wall of the shaft reception means (33), have inner surfaces that extend parallel to the axis of rotation of the rotor (30) and are arranged in opposed relationship with one another, a tip of each of these triangular plates (35, 35') pointing in the direction of the rotor bottom (37).

7. The tube roller pump according to claim 6,
**characterized in that,** at the first position of the rotor (30), the inner surfaces of the triangular plates (35, 35') in the interior of the shaft reception means (33) abut on coplanar side faces (43, 43') in large-area contact therewith, said side faces (43, 43') being formed on two opposed sides of the drive shaft (40).

8. The tube roller pump according to claim 7,
**characterized in that** the second locking element (60) includes a groove (62) and is adapted to be manually moved to a position at which opposed inner surfaces of this groove (62) abut on the side faces (43, 43') of the drive shaft (40) in large area contact therewith.

9. The tube roller pump according to one or more of claims 1 to 8,
**characterized in that** the second locking element (60) is adapted to be pivoted about a pivot shaft (61) within a reception means (34) in the rotor (30), said pivot shaft (61) extending in particular perpendicular to the axis of rotation of the rotor (30) and in spaced relationship therewith.

10. The tube roller pump according to one or more of claims 1 to 9,
**characterized in that,** when occupying the locking position, the first locking element (50) engages a locking geometry (42) in the drive shaft (40).

11. The tube roller pump according to one or more of claims 1 to 10,
**characterized in that** the first locking element (50) is a flat sheet metal part, which is movable radially to the axis of rotation of the rotor and which, at the locking position, engages a circumferentially extending groove (42) of the drive shaft (40).

12. The tube roller pump according to one or more of claims 1 to 11,
**characterized in that** the rotor (30) includes an operating element, in particular a lever or a push-button switch, by means of which the first locking element (50) can be moved manually from the locking position to the release position.

13. A medical device for extracorporeal blood treatment, comprising a tube roller pump including a pump housing (10) having a curved running surface (11) and a rotor (30) that is rotatable within the running surface (11), a tube segment (20) of an extracorporeal blood circuit being adapted to be placed between the running surface (11) and the rotor (30),
**characterized in that**
the tube roller pump is configured according to one or more of claims 1 to 12.

## Revendications

1. Pompe à rouleau tubulaire pour un appareil médical servant au traitement du sang extracorporel, avec un boîtier de pompe (10), qui présente une surface de roulement (11) courbée et un rotor (30) pouvant tourner à l'intérieur de la surface de roulement (11), dans laquelle un segment tubulaire (20) peut être introduit entre la surface de roulement (11) et le rotor (30), et le rotor (30) peut être installé, avec un logement d'arbre (33), sur un arbre d'entraînement (40) de la pompe à rouleau tubulaire, dans laquelle au moins un élément de verrouillage (50 ; 60) est prévu afin de bloquer et/ou de coupler en rotation de manière axiale le rotor (30) sur l'arbre d'entraînement (40), dans laquelle
un premier élément de verrouillage (50) servant à bloquer au choix de manière axiale le rotor (30) sur l'arbre d'entraînement (40) et un deuxième élément de verrouillage (60) servant à coupler en rotation au choix l'arbre d'entraînement (40) et le rotor (30) sont prévus au niveau du rotor (30),
**caractérisée en ce que**
les géométries du logement d'arbre (33) du rotor (30) et de l'arbre d'entraînement (40) sont formées de telle sorte que le rotor (30) peut être guidé dans une première position de rotation relative prédéfinie par rapport à l'arbre d'entraînement (40) lorsqu'il est enfilé de manière axiale sur l'arbre d'entraînement (40), et le premier élément de verrouillage (50) mobile, dans ladite première position, peut être déplacé par l'arbre d'entraînement (40) hors d'une position de verrouillage dans une position de déverrouillage, ce qui permet de déplacer le rotor (30), lorsqu'il continue à être enfilé sur l'arbre d'entraînement (40), dans une deuxième position axialement définie, dans laquelle le premier élément de verrouillage (50) peut être ramené, en particulier de manière automatique, dans la position de verrouillage, et d'amener en prise, en particulier de manière manuelle, le deuxième élément de verrouillage (60) de telle sorte avec l'arbre d'entraînement (40) qu'un couple de rotation peut être transmis de l'arbre d'entraînement (40) sur le rotor (30).

2. Pompe à rouleau tubulaire selon la revendication 1,
**caractérisée en ce que** le premier élément de verrouillage (50) dans la première position peut être déplacé par l'arbre d'entraînement (40) à l'encontre d'une force de ressort hors d'une position de verrouillage dans une position de déverrouillage, tandis que l'élément de verrouillage (50) axial dans la deuxième position peut être ramené par la force de ressort dans la position de verrouillage.

3. Pompe à rouleau tubulaire selon l'une quelconque des revendications 1 ou 2 ou selon les deux,
**caractérisée en ce que** l'arbre d'entraînement (40) et le logement d'arbre (33) présentent respectivement des géométries de glissement (41, 35, 35'), qui glissent les unes contre les autres, quand le rotor (30) est guidé dans la première position par rapport à l'arbre d'entraînement (40) lorsqu'il est enfilé de manière axiale sur l'arbre d'entraînement (40).

4. Pompe à rouleau tubulaire selon la revendication 3,
**caractérisée en ce que** le premier élément de verrouillage (50) dans la première position peut être déplacé, par le contact avec la géométrie de glissement (41) de l'arbre d'entraînement (40), hors de la position de verrouillage dans la position de déverrouillage.

5. Pompe à rouleau tubulaire selon l'une quelconque des revendications 3 ou 4 ou selon les deux,
**caractérisée en ce que** la géométrie de glissement (41) est formée au niveau de l'arbre d'entraînement (40) par une section d'extrémité en forme de coin, moyennant quoi deux surfaces de coin placées par rapport à l'axe de rotation de l'arbre d'entraînement (40) sont réalisées.

6. Pompe à rouleau tubulaire selon l'une quelconque ou selon plusieurs des revendications 3 à 5,
**caractérisée en ce que** la géométrie de glissement du logement d'arbre (33) est formée par deux plaques (35 ; 35') triangulaires, qui sont réalisées au niveau de la paroi intérieure du logement d'arbre (33), qui présentent des surfaces intérieures parallèles par rapport à l'axe de rotation du rotor (30) et qui se font face les unes les autres, dans laquelle une pointe des plaques (35, 35') triangulaires pointe respectivement en direction du fond de rotor (37).

7. Pompe à rouleau tubulaire selon la revendication 6,
**caractérisée en ce que** les surfaces intérieures des plaques (35, 35') triangulaires reposent à plat à l'intérieur du logement d'arbre (33) dans la première position du rotor (30) au niveau de surfaces latérales (43, 43') planes et parallèles les unes par rapport aux autres, lesquelles sont réalisées au niveau de deux côtés se faisant face de l'arbre d'entraînement (40).

8. Pompe à rouleau tubulaire selon la revendication 7,
**caractérisée en ce que** le deuxième élément de verrouillage (60) présente une rainure (62) et peut être amené de manière manuelle dans une position, dans laquelle des surfaces intérieures se faisant face les unes les autres de ladite rainure (62) reposent à plat au niveau des surfaces latérales (43, 43') de l'arbre d'entraînement (40).

9. Pompe à rouleau tubulaire selon l'une quelconque ou plusieurs des revendications 1 à 8,
**caractérisée en ce que** le deuxième élément de verrouillage (60) peut pivoter autour d'un axe de pivotement (61) en particulier perpendiculaire par rapport à l'axe de rotation du rotor (30) et tenu à distance de celui-ci, à l'intérieur d'un logement (34) dans le rotor (30).

10. Pompe à rouleau tubulaire selon l'une quelconque ou plusieurs des revendications 1 à 9,
**caractérisée en ce que** le premier élément de verrouillage (50) dans la position de verrouillage vient en prise avec une géométrie d'enclenchement (42) dans l'arbre d'entraînement (40).

11. Pompe à rouleau tubulaire selon l'une quelconque ou plusieurs des revendications 1 à 10,
**caractérisée en ce que** le premier élément de verrouillage (50) est une pièce en tôle plate pouvant être déplacée de manière radiale par rapport à l'axe de rotation du rotor, laquelle vient en prise, dans la position de verrouillage, avec une rainure (42) s'étendant tout autour dans la direction périphérique de l'arbre d'entraînement (40).

12. Pompe à rouleau tubulaire selon l'une quelconque ou plusieurs des revendications 1 à 11,
**caractérisée en ce que** le rotor (30) présente un élément d'actionnement, en particulier un levier ou un pressostat, avec lequel le premier élément de verrouillage (50) peut être déplacé de manière manuelle hors de la position de verrouillage dans la position de déverrouillage.

13. Appareil médical servant au traitement du sang extracorporel, comprenant une pompe à rouleau tubulaire avec un boîtier de pompe (10), qui présente une surface de roulement (11) courbée et un rotor (30) pouvant tourner à l'intérieur de la surface de roulement (11), dans lequel un segment tubulaire (20) d'un circuit de sang extracorporel peut être introduit entre la surface de roulement (11) et le rotor (30), **caractérisé en ce que** la pompe à rouleau tubulaire est réalisée selon l'une quelconque ou plusieurs des revendications 1 à 12.
